# EUROPEAN PATENT APPLICATION

(11) **EP 1 118 676 A2**
(43) Date of publication of application: **25.07.2001**
(21) Application number: 01101358.8
(22) Date of filing: 22.01.2001
(51) Int. Cl.: C12Q 1/24

(54) **Cell isolation method**

(30) Priority: 21.01.2000 EP 00101173
(71) Applicant: Chemagen AG, 52499 Baesweiler (DE)
(72) Inventor: à Brassard, Lothar, Dr., 52066 Aachen (DE); Oster, Jürgen, Dr., 52134 Herzogenrath (DE)
(74) Representative: Wolff, Felix, Dr.

(57) **Abstract**

The present invention relates to a method of isolating microorganisms present in a sample, in particular to a method of isolating bacteria present in a sample.

## Description

The present invention relates to a method of isolating microorganisms present in a sample, in particular to a method of isolating bacteria present in a sample.

The majority of current techniques, which seek to analyse samples qualitatively, and quantitatively for the presence of microorganism are directed to identification of a specific microorganism, e.g. a pathogen. Immunomagnetic separation of strains of Listeria and Salmonella from food and clinical samples has been described (see for example Skjerve et al. Appl. Environ. Microbiol. 1990, 3478-3481). Such techniques rely upon the use of ligands, generally antibodies, specific for the microorganism concerned. As it relies upon the use of specific ligands, immunomagnetic separation is applicably only in situations where a particular microorganism is sought to be identified and not where a general screen for microorganism, which may be present, is desired. Immunomagnetic separation will not really pick up unsuspected microorganisms.

There are situations, for example when analysing environmental samples, such as water samples where it may be desirable to obtain an estimate of the total concentration of microorganisms present. Limits may be placed on the total amount of bacteria, which may be present in the water supply, which is independent of concerns about the presence of particular bacteria. Concentration of bacteria e.g. in a river or lake may provide useful information about the state of that body of water, levels of nutrients, run-off of agrochemicals etc.

Methods currently used to obtain estimates of the amount of microorganisms present in a sample or to isolate the microorganisms from the sample for further analysis rely on rather crude separation techniques involving filtration and/or centrifugation and culturing on agar plates. These methods may take several days and are rather complex and imprecise.

There is therefore a need for a method, which is able to isolate a number of different species of microorganisms from a sample, preferably in a single step, which is quick and convenient to perform.

The present invention addresses these requirements and thus according to one aspect of the present invention is provided a method isolating microorganisms from a sample which method comprises binding said microorganisms to a solid support by means of a non-specific ligand immobilised on said solid support.

The present invention therefore provides, through utilisation of the interactions between non-specific ligands attached to solid supports and binding partners for said ligands (receptors) found on the surface of microorganisms, a general separation method which allows for the capture of broad classes of microorganism. As the essence of the present invention is the non-specific interaction between the immobilised ligand and microorganisms, when reference is made herein to "isolating microorganisms" it is intended to describe the isolation of microorganisms in a non-specifies and non-genus specific manner. In other words, the immobilised ligand is able to bind a several genera of microorganism, at least 2 different genera, preferably 3 or more, more preferably at least 5 or 7 different genera, most preferably at least 10 or even 14 or more different genera.

The microorganisms are "isolated" in that they are bound to the solid support and then may be separated from the remainder of the sample by removing the solid support with microorganisms bound thereto or by removing, e.g. by running off, the reminder of the sample. Where the solid support is magnetic, manipulation of the support/microorganism complex is especially convenient.

The method of the present invention may be used to isolate a wide variety of microorganisms, including the protests, algae, protozoa and fungi as well as mycoplasmas and all bacteria and viruses The methods of the invention are particularly suitable for isolating bacteria. Bacterial classification is a complex and ever changing science but 'true' bacteria (eubacteria) may be divided into a number of Parts (Bergey's Manual of Determinative Biology) e.g. phototrophic bacteria, gliding bacteria, sheathed bacteria and Gram-Positive locci. Each Part may in turn be divided into one or more orders which families and genera within that order. Bacteria from more than one taxonomic genera, order or family or even Part may be readily isolated using the methods of the present invention. Thus effective separation from a sample of bacteria from some or all of the following families and genera of bacteria may be achieved using the present methods: Aeromonas, Bacillus, Campylobacter, Citrobacter, Clostridium, Enterobacter, E. coli (pathogen and non-pathogen), Hafnia, Klebsiella, Listeria, Proteus, Salmonella, Shewanella, Serratia, Shigella, Vibrio, Yersinia.

In certain circumstances, the sample may only contain a limited number of types of bacteria and the method may only result in a few species of bacteria, or even only one or two sample. Such a method is still not a specific isolation method as the immobilised ligand is capable of binding to a wide variety of bacteria even if the available bacterial populations are rather limited. The fact that a specific solid-support-ligand complex does not need to be selected in order to perform a given isolation method provides a significant advantage in terms of flexibility and cost as one solid support with attached ligand can be used in a wide variety of isolation/separation methods. It is therefore the general binding capabilities of the solid support, which is particularly advantageous.

Preferably, the methods of the invention will result on a large proportion of the bacteria present in the sample being isolated, both in terms of the proportion of all bacterial cells present and the proportion of types of bacteria. Thus, preferably at least 30%, more preferably at least 50%, most preferably at least 70 or 80% of the bacteria in the sample will be bound to be solid support Alternatively viewed, representatives from at least 20% of the different bacterial species will preferably be isolated, more preferably at least 30 or 40%, most preferably at least 50%, particularly at least 60 or 70%

The 'non-specific' ligand will be one which, as discussed above, is capable of binding more than one bacterial genus, preferably to more than 2 or 3, more preferably to more than 5 or 7 e.g. more than 10 or 14 different genera. There is an interaction between the ligand and its binding partner(s) on the surface of the microorganism, which is responsible for binding, it is not the case that there is simply a general attraction or association between the cells bound by precipitation. The non-specific character of the ligand refers not to the fact that it is capable of binding of associating indiscriminately which moieties on the surface of microorganisms but that its binding partner(s) is not to a certain type of microorganism. As the binding partner is relatively widely found amongst different microorganisms, in particular amongst different genera of bacteria, a rather general and thus non-specific isolation method is provided. Thus the ligand, while it may have one or more specific binding partners, is 'non-specific' in the sense that it is capable of binding to a variety of different bacteria, all of which carry the necessary binding partner.

The ligand is non-proteinaceous and thus antibodies and derivatives and fragments thereof are excluded. In contrast to the non-specific ligands of the present method, such proteinaceous ligands are capable of very specific (i.e. genera, in particular species specific) interactions e.g. with proteins on cell surfaces.

Preferred ligands are carbohydrates including monosaccharides, oligosaccharides (including disaccharides and trisaccharides) and polysaccharides. Suitable monosaccharides include hexoses and pentoses in pyranose and furanose from where appropriate, as well as sugar derivatives such as aldonic and uronic acids and deoxy or amino sugars and sugar alcohols. Suitable monosaccharides may be exemplified by mannose (e.g. D-mannose), galactose (e.g. D-galactose), glucose (e.g. D-glucose), fructose, fucose (e.g. L-fucose), N-acetyl-glucosamine, N-acetylgalactosamine, rhamnose, galactosamine, glucosamine (e.g. D-glucosamine), galacturonic acid, glucuronic acid, N-acetylneuraminic acid, methyl D-mannopyranoside (mannoside), α-methyl-glucoside, galactoside, ribose, xylose, arabinose, saccharate, mannitol, sorbitol, inositol, glycerol and derivatives of the monomers.

Particularly preferred are ogliosaccharides and polysaccharides, which are polymers of monosaccharide monomers, for example polymers incorporating the monosaccharide monomers, discussed above.

Oligosaccharides comprise 2 to 12, preferably 4 to 8, covalently linked monosaccharide units which may be the same or different and which may be linear or branched, preferably branched, e.g. oligomannosyl having 2 to 6 units, maltose, sucrose, trehalose, cellobiose, and salicin. A method for production of oligosaccharides is described in Pen et a. Infection and Immunity (1997), 4199-4206.

Polysaccharides comprise 13 or more covalently linked monosaccharide units which may be the same or different and which may be linear or branched, preferably branched. Suitable polysaccharides will be rich in mannose, galactose and fructose e.g. the galactomannan polysaccharide locust bean gum (referred to herein as GUM 1) (Sigma G-0753) which consist of a mannose backbone with galactose units that branch off regularly every forth mannose unit. This branching differentiates locust bean gum from the very similar compound guar gum, which is also very suitable for coupling. Further polysaccharides include Gum Arabic, Xanthan Gum, Karaya Gum or Konjac Gum.

The inventors have found that ligands based on molecules which are nutrients for microorganisms, such as carbohydrates, provide suitable separations means. It was an aim of the present method to provide an isolation system which as well as utilising receptor/ligand interactions took advantage of nutrients in the media in order to enhance capture of microorganisms. Further nutrients for microorganisms, which may thus be used as non-specific ligands according to the methods of the present invention, include vitamins such as nicotinic acid, riboflavin, thiamine, pyridoxine, pantothenic acid, folic acid, biotin and cobamide and iron-chelating molecules/compounds such as hemin, lactoferrin, transferrin, haemoglobin, aerobactin, ferrichrome (Sigma P8014), ferrienterochelin, enterobactin, ferrixanine,

A solid support having immobilised thereon a ligand, which is capable of binding to microorganisms in a non-specific manner thus, constitutes a further aspect of the present invention. 'Non-Specific' is as defined above and suitable ligands are described herein with reference to the methods.

In the context of investigating the fucose-sensitive and mannose-sensitive hemagglutination of the bacteria *Vibrio cholerae*, L-fucose and D-mannose have been covalently attached to agarose beads (Sanchez et al APMIS (1990) 98 353-357) and thus these solid support-ligand complexes are not included within the scope of the present invention. This previous work was investigating a very specific interaction involving just one species of bacteria and is not a general bacterial isolation method. Therefore the use of these bead-ligand complexes in the context of the methods defined herein do comprise an aspect of the present invention.

The immobilised ligand will preferably be an oligo- or polysaccharide, vitamin (e.g. one required as a nutrient for microorganisms) or iron-chelating compound, polysaccharides being particularly preferred. Examples of particularly preferred ligands are discussed previously in the discussion relating to the methods.

The sample from which microorganisms may be isolated includes environmental samples such as water samples, e.g. from lakes, rivers, sewage plants and other water-treatment centres or soil samples. The methods are of particular utility in the analysis of food samples and generally in health and hygiene applications where it is desired to monitor bacterial levels, e.g. in areas where food is being prepared. Milk products for example may be analysed for Listeria. Conventional techniques for bacterial isolation using immobilised antibodies have proved to be much less effective than our methods for isolating lysteria using non-specific ligands, possibly due to the hydrophobic nature of the immobilised antibody.

Food samples may be analysed by first homogenising where necessary (if a solid sample) the mixing with a suitable incubation media (e.g. peptone water) and incubating at 37°C overnight. Food such as cheese, ice cream eggs, wheat floor, rolled oats, boiled rice, peppers, tomato, broccoli, beans, peanuts and marzipan may be analysed in this way.

Samples from which microorganisms may be isolated according to the presents method may be clinical samples taken from the human or animal body. Suitable samples include, whole blood and blood derived products, urine, faeces, cerebrospinal fluid or any other body fluids as well as tissue samples and samples obtained by e.g. a swab of a body cavity.

The sample may also include relatively pure or partially purified starting materials, such as semi-pure preparations obtained by other cell separation processes.

Suitable solid supports for use in the present invention may be any of the well know supports or matrices which are currently widely used or proposed for immobilisation, separation etc. These my take the form of particles, sheets, gels, filters, membranes, fibres, capillaries, or microtitre stripe, tubes, plates or wells etc.

Conveniently the support may be made of glass, silica, latex or a polymeric material. Preferred are materials presenting a high surface area for binding of the cells, and subsequently, of the nucleic acid. Such supports will generally have an irregular surface and may be for example be proud or particulate e.g. particles, fibres, webs, sinters or sieves. Particulate materials e.g. beads are generally preferred due to their greater binding capacity, particularly polymeric beads.

Conveniently, a particulate solid support used according to the invention will comprise spherical beads. The size of the beads is not critical, but the may for example be of the order or diameter of at least 1 and preferably at least 2 µm, and have a maximum diameter of preferably not more than 10 and more preferably not more than 6 µm. For example, beads of diameter 2.8 µm and 4.5 µm have been shown to work well.

Monodisperse particles, that is those which are substantially uniform in size (e.g. size having a diameter standard deviation of less than 5%) have the advantage that they provide very uniform reproducibility of reaction. Monodisperse polymer particles produced by the technique in US-A-4336173 are especially suitable.

Non-magnetic polymer beads suitable for use in the method of the invention are available from Dyno Particles AS (Lillestrom, Norway) as well as from Qiagen, Pharmacia and Serotec.

However, to aid manipulation and separation, magnetic beads are preferred. The Term "magnetic" as used herein means that the support is capable of having a magnetic moment imparted to it when placed in a magnetic field. In other words, a support comprising magnetic particles may readily be removed by magnetic aggregation, which provides a quick, simple and efficient way of separating the particles following the cell and nucleic acid binding steps, and is a far less rigorous method than traditional techniques such as centrifugation which generate shear forces which may disrupt cells or degrades nucleic acids.

Thus, using the method of the invention, the magnetic particles with cells attached may be removed onto a suitable surface by application of a magnetic field e.g. using permanent magnet. It is usually sufficient to apply a magnet to the side of the vessel containing the sample mixture to aggregate the particles to the wall of the vessel and to pour away the remainder of the sample.

Especially preferred are superparamagnetic particles for example those described by Sintef in EP-A-106873, as magnetic aggregation and clumping of the particles during reaction can be avoided, thus ensuring uniform and nucleic acid extraction.

The well-know magnetic particles sold by Dynal AS (Oslo, Norway) as DYNABEADS are suited to use in the present invention.

Functionalised coated particles by modification of the beads according to US patens 4,336,173, 4,459,378 and 4,654,267. Thus, beads, or other supports, may be prepared having different types of functionalised surface, for example positively or negatively charged, hydrophilic or hydrophobic.

Particularly preferred particles for use as a solid support in the methods of the invention are spherical shaped in the methods of the invention are spherical shaped polymer particles (beads) based on PVA (polyvinyl alcohol) in which a magnetic colloid has been encapsulated. These beads may be produced through suspension of a polymer phase containing an emulsifier as described in CA 2,227,608. The particles, which may vary in size from 1-8 µm, preferably are available from chemagen AG, Germany .

Appropriate buffers etc. may be used as media for the isolation to achieve conditions appropriate for binding. Conveniently, a buffer of appropriate charge, to etc, may be added to the sample prior to, simultaneously with or after contact with the solid support. PBS is a suitable cell binding buffer.

Methods for attachment of a ligand to a solid support are well known in the art. Typically, the solid support will first be activated and the reacted with the ligand, the ligand may itself have been modified slightly for covalent attachment to the solid support. In the case of the superparamagnetic beads from chemagen, which are discussed above, the polyvinyl alcohol matrix may be activated by the introduction of isocyanate functionalities via an 8-atom spacer. These activated beads (M-PVA Ak2) can the be used for the direct coupling of molecules containing amino or hydroxy functionalities. A typical coupling reaction would be as follows:

In order to investigate cell binding or in certain applications of the present invention where information about specific microorganisms is required, it may be desirable after the cells have bound to the solid support to isolate nucleic acid from the microorganism-beads complex and analyse it, e.g. by PCR. Thus, after the microorganisms have bound to said solid support where will be a cell lysis step to release the nucleic acid from microorganisms for subsequent analysis. The released nucleic acid may be analysed in solution but is more conveniently analysed after it has bound to a solid support, this solid support may be the same or different but is preferably the same as the solid support to which the microorganisms themselves bound. Thus, in a further aspect, the present invention provides a method of isolating nucleic acid from microorganisms present in a sample said method comprising:
a) binding said microorganisms to a solid support by means of a non-specific ligand immobilised on said solid support
b) lysing the microorganisms; and optionally
c) binding nucleic acid released from said lysed microorganisms to a solid support

Suitable methods for lysing the microorganisms, binding the nucleic acids thus released and analysing the nucleic acid are provided in WO98/51693 which is incorporated herein by reference.

The nucleic acid may be DNA, RNA or any naturally occurring or synthetic modifications thereof, and combination thereof. Preferably however the nucleic acid will be DNA, which may be single or double stranded or in any other form, e.g. linear or circular.

Following binding, the isolated or support-bound microorganism, are lysed to release their nucleic acid. Methods of cell lysis are well known in the arte and widely described in the literatures and any of the known methods may be used. Different methods may be more appropriate for different microorganisms, but any of the following methods could, for example, be used: detergent lysis using e.g. SDS, LIDS or sarkosyl in appropriate buffers; the use of chaotropes such as Guanidium Hydrochloride (GHCI), Guanidium thiocyanate (GTC), sodium iodide (Nal) perchlorate etc; mechanical disruption, such as by a French press, sonication, grinding with glass beads, alumina or in liquid nitrogen; enzymatic lysis, for example using lysozyme, proteinases, pronases or cellulases or any of the other lysis enzymes commercially available; lysis of cells by bacteriophage or virus infection; freeze drying; osmotic shock; microwave treatment; temperature treatment; e.g. by heating or boiling, or freezing, e.g. in dry ice or liquid nitrogen, and thawing; alkaline lysis. As mentioned above, all such methods are standard lysis techniques and are well known in the art, and any such method or combination of methods may be used.

Conveniently, lysis may be achieved according to the present invention by using chaotropes and/or detergents. For example, in the case of bacterial cells, the combination of a chaotropes with a detergent exemplary suitable lysis agent thus includes a chaotropes such as GTS or GHCI and a detergent such as SDS or Sarkosyl. The lysis agents may be supplied in simple aqueous solution, or they may be included in a buffer solution, to form a so-called "lysis buffer". Andy suitable buffer may be used, including for example Tris, Bicine, Tricine and prospate buffers. Alternatively the lysis agents may be added separately. Suitable concentrations and amounts of lysis agents will vary according to the precise system, nature of the cells etc. and may be appropriately determined, but concentrations of e.g. 2 M to 7 M chaotropes such as GTC, GHCI, NAI or perchlorate may be used, 0.1M to 1 M alkaline agents such as NaOH, and 0.1 to 50% (w/v) e.g. 0.5 to 15% detergent. Thus, and example of a suitable representative lysis buffer includes an aqueous solution of 4 M GTC, 1% (w/v) sarkosyl.

The isolated, support-bound microorganisms may conveniently be removed or separated from the remainder of the sample, thereby concentrating or enriching the cells. Thus the cell-binding step serves to enrich the cells or to concentrate them in a smaller volume than the initial sample. Lysis then may conveniently be achieved by adding an appropriate lysis buffer containing the desired lysis agents or by subjecting the isolated cells to the desired lysis conditions. For example, in the case of simple adding a lysis buffer containing appropriate lysis agents, the isolating cells may simply be incubated in the presence of the lysis buffer for a suitable interval to allow lysis to take place. Different incubation conditions may be appropriate for different lysis systems, and are known in the art. For example for a detergent and /or chaotropes containing lysis buffer, incubation may take place at room temperature or at higher temperatures e.g. 37°C or 65°C. Likewise, time of incubation may be varied from a few minutes e.g. 5 or 10 minutes to hours, e.g. 1 to 2 hours. In the case of GTC/sarkosyl lysis buffers and bacterial cells, incubation at e.g. 65°C for 10-20 minutes has been found to be appropriate, but this may of course be varied according to need. For enzymatic lysis, e.g. using proteinases K etc, longer treatment times may be required, e.g. overnight.

Following lysis, the released nucleic acid is conveniently bound to a solid support, preferably the one which the lysed microorganisms are bound. Although, for example, a mixed population of beads may be provided some with a non-specific ligand for binding to microorganisms and others adapted to bind nucleic acid.

This nucleic acid binding may be achieved in any way known in the art of binding nucleic acid to a solid support. Conveniently, the nucleic acid is bound non-specifically to the support i.e. independently of sequence. Thus, for example the released nucleic acid may be precipitated onto the support using any of the know precipitants for nucleic acid, e.g. alcohols, alcohol/salt combinations, polyethylene glycols (PEGs) etc. Precipitation of nucleic acids onto beads in this manner is described for example in WO 91/12079. Thus, salt may be added to the support and released nucleic acid in solutions, followed by addition of alcohol, which will cause the nucleic acid to precipitate. Alternatively, the salt and alcohol may be added together, or the salt may be omitted. As described above in relation to the cell-binding step, any suitable alcohol or salt may be used, and appropriate amounts or concentrations may readily be determined.

Alternative non-specific nucleic acid-binding techniques include the use of detergents as described in WO 96/18731 of Dynal AS (the so-called "DNA Direct" procedure), and the use of chaotropes and a nucleic acid-binding solid phase such as silica particles as described by Akzo N.N. in EP-A-0389063.

Ionic binding of the nucleic acid to the support may be achieved by using a solid support having a charged surface, for example a support coated with polyamines.

The support which is used in the method of the invention may also carry functional groups which assist in the specific or non-specific binding or nucleic acids, for example DNA binding proteins e.g. leucine zippers or histones or intercalating dyes (e.g. ethidium bromide or Hoechst 42945) which may be coated onto the support.

Likewise, the support may be provided with binding partners to assist in the selective capture of nucleic acids. For example, complementary DNA or RNA sequences, or DNA binding proteins may be used, or viral protein binding to viral nucleic acid. The attachment of such proteins to the solid support may be achieved using techniques well known in the art.

A convenient method of precipitating the nucleic acid according to the invention is by adding a precipitant, e.g. alcohol, to the mixture containing the support and lysed cells. Thus, an appropriate volume of alcohol, e.g. 100% or 96% ethanol, may simply be added to the mixture, and incubated for a time period sufficient to allow the released nucleic acid to become bound to the support. The incubation conditions for this step are not critical and may simply comprise incubating at 5-10 minutes at room temperature. However, the length of time may be varied, and temperature increased according to choice.

Although not necessary, it may be convenient to introduce one ore more washing steps to the isolation method of the invention, for example following the nucleic acid binding step. Any conventional washing buffers or other media may be used. Generally speaking, low to moderate ionic strength buffers are preferred e.g. 10 mM Tris-HCI at ph 8.0/10mM NaCl. Other standard washing media, e.g. containing alcohols, may also be used, if desired, for example washing 70% ethanol.

The use of magnetic particles permits easy washing steps simply by aggregating the particles, removing the nucleic acid binding medium, adding the washing medium and reaggregating the particles as many times as required.

Following the nucleic acid isolation process and any optional process and any optional washing steps which may be desired, the support carrying the bound nucleic acid may be transferred e.g. resuspended or immersed into any suitable medium e.g. water or low ionic strength buffer. Depending on the support and the nature of any subsequent processing desired, it may or may not be desirable to release the nucleic acid from the support.

In the case of a particulate solid support such as magnetic or non-magnetic beads, this may in many cases be used directly, for example in PCR or other amplifications without eluting the nucleic acid from the support. Also for man y DNA detection or identification methods elution is not necessary since although the DNA may be randomly in contact with the bead surface and blind at a number of points by hydrogen bonding or ionic or other forces, there will generally be sufficient lengths of DNA available for hybridisation to oligonucleotides and for amplification.

However, if desired, elution of the nucleic acid may readily be achieved using known means, for example by heating, e.g. to 65°C for 5 to 10 minutes, following which the support may be removed from the medium leaving the nucleic acid in solution. Such heating is automatically obtained in PCR by the DNA denaturation step preceding the cycling program.

If it is desired to remove RNA from DNA, this may be achieved by destroying the RNA before the DNA separation step, for example by addition of an RNAase or an alkali such as NaOH.

An advantage of the present invention is that it is quick and simple to perform, and the simplicity of the method allows for high throughput of samples.

The invention is advantageously amenable to automation, particularly if particles, and especially, magnetic particles are used as the support.

As mentioned above, the method of the invention has particular utility as a preliminary first step to prepare nucleic acid for use in nucleic acid-based detection procedures.

As mentioned above, advantageously bound nucleic acid need not be eluted or removed from the support prior to carrying out the detection step, although this may be performed if desired. Whether or not the nucleic acid is eluted may also depend on the particular method, which was used in the nucleic acid binding step. Thus certain nucleic acid-binding procedures will bind the nucleic acid more tightly than others. In the case of DNA-binding using detergents (e.g. by DNA direct) for example, the nucleic acid will elute from the solid support when an elution buffer or other appropriate medium is introduced. Nucleic acid bound by means of a precipitant such as alcohol or a chaotropes will remain more tightly bound may not elute when placed in a buffer medium, and may require heating to be eluted.

Thus, the support-bound nucleic acid may be used directly in a nucleic acid based detection procedure, especially if the support is particulate, simply by resuspending the support in, or adding to the support, a medium appropriate for the detection step. Either the nucleic acid may elute into the medium, or as mentioned above, it is not necessary for it to elute.

A number of different techniques for detecting nucleic acids are known and described in the literature and any if these may be used according to the present invention. At its simplest the nucleic acid may be detected by hybridisation to a probe and very much such hybridisation protocols have been described (see e.g. Sambrook et al., 1989, molecular Cloning: A Laboratory Manual, 2^{nd} Ed. Cold Spring Harbor Press, Cold Spring Harbor, NY) Most commonly, the detection will involve an *in situ* hybridisation step, and/or an *in vitro* amplification step using any of the methods described in the literature for this. Thus, as mentioned, techniques such as LAR, 3SR and the Q-beta-replicase system may be used. However, PCR and its various modifications e.g. the use of nested primers, will generally be the method of choice (see e.g. Abramson and Myers, 1993, Current Opinion in Biotechnology, 4: 41-47 for a review of nucleic acid amplification technologies)

Other detection methods may be based on a sequencing approach, for example, the minisequencing approach as described by Syvänen and Söderlund, 1990, Genomics, 8: 684-692.

In amplification techniques such as PCR, the heating required in the first step to melt the DNA duplex may release the bound DNA from the support. Thus, in the case of a subsequent detection step, such as PCR, the support bound nucleic acid may be added directly to the reaction mix, and the nucleic acid will elute in the first step of the detection process. The entire isolated support bound nucleic acid sample obtained according to the invention may be used in the detection step, or an aliquot.

The results of the PCR or other detection step may be detected or visualised by many means, which are described in the art. For example the PCR or other amplification products may be run on an electrophoresis gel e.g. an ethidium bromide stained agarose gel using know techniques. Alternatively, the DIANA system may be used, which is a modification of the nested primer technique. In the DIANA (Detection of Immobilised Amplified Nucleic Acid) system (see Wahlberg st. al., Mol. Cell Probes 4: 285(1990)), the inner, second pair of primers carry, respectively, means for immobilisation to permit capture of amplified DNA, and a label or means for attachment of a label to permit recognition. This provides the dual advantages of a reduced background signal, and a rapid and easy means for detection of the amplified DNA.

The amplified nucleic acid may also be detected, or the result confirmed, by sequencing, suing any of the many different sequencing technologies which are now available, e.g. standard sequencing, solid phase sequencing, cyclic sequencing, automatic sequencing and minisequencing.

The various reactants and components required to perform the methods of the invention may conveniently be supplied in kit form. Such kits represent a further aspect of the invention.

At its simplest, this aspect of the invention provides a kit for isolating microorganisms from a sample comprising:
a) a solid support having immobilised thereon a ligand which allows non-specific binding to microorganisms;
b) means for binding microorganisms to said solid support optionally
c) means for lysing said cells; optionally
d) means for binding nucleic acid released from said lysed cells to a solid support

The various means (b), (c) and (d) may be as described and discussed above, in relation to the method of the invention.

A further optional component is (e), means for detecting the presence or absence of nucleic acid characteristic of a target microorganism. As discussed above, such means may include appropriate probe or primer oligonucleotide sequence for use in hybridisation and/or amplification-based detection techniques.

Optionally further included in such a kit may be buffers, salts, polymer, enzymes, etc.

The invention will now be described in more detail with the following non-limiting Examples:

### Example 1

Immobilisation of saccharide to M-PVA magnetic beads using the isocyanate activation method

For the immobilisation of saccharides, the hydroxyl functions at the surface of M-PVA magnetic beads (M-PVA 012; chemagen AG, Germany) were activated using hexamethylene-diisocyanate (HMDI, Fluka, Germany).
a) Bead-OH + OCN-(CH₂)₆-NCO → Bead-O-C(O)NH-(CH₂)₆-NCO
b) Bead-O-C(O)NH-(CH₂)₆-NCO + saccharide-OH → Bead-O-C(O)NH-(CH₂)₆-NC(O)-O-saccharide
1 g of M-PVA 012 were reacted under an argon atmosphere with 250 µl HMDI in 10 ml dry Dimethylsulfoxide (Fluka, Germany) for 1 hour at room temperature. The isocyanate-activated magnetic beads were then separated magnetically, the supernatant was discarded and the beads were washed once with 10 ml Dimethylsulfoxide. A solution of Gum locust bean (0,1 M in water; Sigma, Germany) was added to the beads and mixed for 12 hours. The saccharide coated beads were then washed 5 times with water.

With this method polysaccharides other than can also be immobilised successfully: for example Xanthan Gum, Guar Gum, Karaya Gum, Gum Arabic or Konjac Gum. For the subsequent binding of bacteria, poysaccharides containing many mannose units together with galactose or glucose units are most useful.

### Example 2

Immobilisation of saccharides to carboxylated M-PVA magnetic beads using an activated ester method

For the immobilisation of saccharides, the carboxyl functions at the surface of M-PVA magnetic beads (M-PVA C22; chemagen AG, Germany) were activated using water soluble carbodiimide EDC (Sigma, Germany) and the N-Hydroxysuccinimide (NHS, Fluka, Germany).
a) a) Bead-COOH + EDC/NHS-OH → Bead-C(O)-O-NHS
b) b) Bead-C(O)-O-NHS + saccharide-OH → Bead-C(O)-O-saccharide

1 g of M-PVA C22 was reacted with 120 mg EDC and 24 mg NHS in 12 ml 0,1 M MES-buffer pH 6,5 for 1 hour at room temperature. The activated beads were separated magnetically and the supernatant was discarded. A solution of Gum locust bean (0,1 M in water; Sigma, Germany) was added to the activated beads and the suspension was mixed for 5 hours at room temperature. The resulting saccharide coated beads were then washed 3 times with 0,1 N sodium hydroxide and 2 times with water.

### Example 3

Testing of the cell binding properties using the saccharide coated M-PVA magnetic beads
1 mg of saccharide coated M-PVA magnetic beads, according examples 1 and 2, were each incubated with 1 ml of different bacterial cultures, either with pure undiluted cultures (o.n.) or cultures diluted in water or a buffer (PBS) for 5 minutes to 15 hours. The beads were then separated magnetically, the supernatant was discarded and the beads were washed once with 1 ml PBS and then resuspended in 1 ml PBS.

In order to investigate cell binding, different dilutions of the resulting suspension were plated on agar-plates (e.g. LB plates) and grown for 15-40 hours. The number of viable bacteria bound to the beads was determined by counting.

Additionally, 100 µl of each bead suspension was incubated with 200 µl lysis buffer (lysis buffer from the M-PVA DNA200 kit from chemagen AG, Germany) for 5 minutes at room temperature. 500 µl of binding buffer (binding buffer from the M-PVA DNA200 kit from chemagen AG, Germany) was then added and the resulting suspension incubated for another 5 minutes at room temperature. After magnetic separation, the supernatant was discarded and the beads washed according to the protocol of the M-PVA DNA200 kit. The bacterial DNA could be used while bound to the beads directly or following elution from the beads with 10 mM Tris-buffer pH 8. The PCR was performed using specific primer sets typical for the bacteria used.

### Example 4

### Binding of bacteria from milk using the saccharide coated M-PVA magnetic beads

1 µl of a overnight culture of E-coli bacteria in LB culture medium were added to 10 ml milk. 100 µl of this mixture was added to a solution of 400 µl LB culture medium, and 400 µl PBS containing 200 pg of saccharide coated M-PVA magnetic beads and incubated for 3 hours at 37 °C. The beads together with the bound bacteria were then separated magnetically, the supernatant was discarded and the beads were washed once with 1 ml PBS and then resuspended in 1 ml PBS.

In order to investigate the binding of E-coli bacteria to the beads, different dilutions of the resulting suspension were plated on agar-plates (e.g. LB plates) and grown for 15-40 hours. The number of viable bacteria bound to the beads was determined by counting.

Additionally, 100 µl of the E-coli containing bead suspension were incubated with 200 µl lysis buffer (lysis buffer from the M-PVA DNA200 kit from chemagen AG, Germany) for 5 minutes at room temperature. Then 500 µl of binding buffer (binding buffer from the M-PVA DNA200 kit from chemagen AG, Germany) was added and the resulting suspension incubated for another 5 minutes at room temperature. After magnetic separation, the supernatant was discarded and the beads washed according to the protocol of the M-PVA DNA200 kit. The bacterial DNA could then be used bound directly to the beads or after elution from the beads with 10 mM Tris-buffer pH 8. PCR was performed using a primer set specific for the malB region of the E-coli DNA according to the literature (U. Candrian et al., Int. J. Food Microbiol. 12 (1991) 339-352).

### Example 5

### Detection of bacterial DNA using saccharide coated M-PVA together with DNA-binding M-PVA magnetic beads

The binding of E-coli bacteria was done as described in example 4.

For the specific determination of E-coli DNA, 100 µg of M-PVA DNA magnetic beads (from the M-PVA DNA200 kit; chemagen AG, Germany) in 500 µl binding buffer (M-PVA DNA200 kit) were to the lysate and incubated for 5 minutes at room temperature. Following magnetic separation, the supernatant was discarded and the beads washed according to the protocol of the M-PVA DNA200 kit. The bacterial DNA could be used while bound to the beads directly or following elution from the beads with 10 mM Tris-buffer pH 8. The PCR was performed using a primer set specific for the malB region of the E-coli DNA according to the literature (U. Candrian et al., Int. J. Food Microbiol. 12 (1991) 339-352).

Using this method the detection sensitivity could be increased

## Claims

1. A method for isolating microorganisms unspecifically using magnetic or non-magnetic beads based on Polyvinylalcohol (PVA-beads), said method comprising the steps of:
a. treating the PVA-beads with a mixture containing microorganisms;
b. adding an appropriate buffer for the binding of microorganisms to the PVA-beads and incubating for a period of time dependent on the nature of the micro-organisms;
c. separating the PVA-beads with the bound microorganisms and discarding the supernatant;
d. optionally, washing the microorganisms bound to the magnetic PVA-beads.

2. The method of claim 1 wherein the PVA-beads are covalently coated with saccharides, which can be monosaccharides, oligosaccharides and preferably polysaccharides.

3. The method of claim 1 wherein the PVA-beads are non-covalently coated with saccharides, which can be monosaccharides, oligosaccharides and preferably polysaccharides.

4. The method of claim 1 wherein the microorganisms can be viruses, fungi or preferably bacteria.

5. The method of claim 1 wherein the mixtures contain food, clinical samples or culture media.

6. The method of claim 1 wherein the buffer for binding is a buffer of appropriate ionic concentration such as Phosphate-buffered saline (PBS) or a appropriate Tris-buffer.

7. The method of claim 1 wherein the PVA-beads with the bound microorganisms are separated magnetically (using a permanent or electro magnet, which is placed outside or inside of the reaction tube).

8. The method of claim 1 wherein the PVA-beads with the bound microorganisms are separated by centrifugation or filtration methods.

9. The method of claim 1 wherein the microorganisms are bound directly from the crude mixture.

10. The method of claim 1 wherein the microorganisms are bound from a culture after a pre-culturing step.

11. The method of claim 10 wherein the culture medium for the pre-culturing step is mixed with the PVA-beads and optionally an appropriate buffer for the binding of microorganisms.

12. The method of claim 1 wherein after point c or, optionally d, the immobilised microorganisms are lysed using an appropriate lysis buffer.

13. The method of claim 12 wherein after lysis an appropriate binding buffer is added and the nucleic acid is bound to the same PVA-beads that were used for the binding of the microorganisms.

14. The method of claim 12 wherein after lysis an appropriate binding buffer and other nucleic acid binding PVA-beads are added, and the nucleic acid is bound to the PVA-beads which are different from those that were originally used for the binding of the microorganisms.
